(19) Europäisches Patentamt
European Patent Office
Office européen des brevets

(11) **EP 4 046 648 A1**

(12) **EUROPEAN PATENT APPLICATION**
published in accordance with Art. 153(4) EPC

(43) Date of publication:
**24.08.2022 Bulletin 2022/34**

(21) Application number: **20873458.2**

(22) Date of filing: **09.10.2020**

(51) International Patent Classification (IPC):
**A61K 38/17** (2006.01)   **A61P 25/28** (2006.01)

(52) Cooperative Patent Classification (CPC):
**A61K 38/17; A61P 25/28**

(86) International application number:
**PCT/CN2020/000239**

(87) International publication number:
**WO 2021/068432 (15.04.2021 Gazette 2021/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.10.2019 CN 201910986752**
**12.04.2020 CN 202010298240**

(71) Applicant: **Qi, Zhankai**
**Shanghai 200065 (CN)**

(72) Inventors:
• **QI, Hyatt**
**Shanghai 200065 (CN)**
• **QI, Zhankai**
**Shanghai 200065 (CN)**

(74) Representative: **Sun, Yiming**
**HUASUN Patent- und Rechtsanwälte**
**Friedrichstraße 33**
**80801 München (DE)**

(54) **APPLICATION OF ELAPIDAE SNAKE POSTSYNAPTIC NEUROTOXIN MONOMER MOLECULE IN TREATMENT OF ALZHEIMER'S DISEASE**

(57) Alzheimer's disease is a senile disease in which the main clinical manifestation is progressive memory impairment, thinking, reasoning and movement impairment. The pathological manifestation of Alzheimer's disease is a degenerative process of the nervous system characterized by diffused brain atrophy which is related to the inflammatory response of brain tissue. Therefore, down-regulating inflammatory cytokines and inhibiting inflammatory response can be an important means to improve the pathological basis and clinical symptoms of Alzheimer's disease. By conducting Morris water maze tests on experimental rats and measuring the inflammatory cytokines in the brain tissue thereof, the elapidae snake postsynaptic neurotoxin monomer molecule was found to be able to inhibit the inflammatory cytokines in the brain tissue of rats with senile dementia and improve the learning and memory ability of the rats with senile dementia, providing a possibility for the application of the Elapidae snake postsynaptic neurotoxin monomer molecule in the treatment of Alzheimer's disease.

SEQUENCE LISTING

<110> qi, zhankai

<120>   Use of elapidae neurotoxin for the treatment of Alzheimer's Disease

<130>   ALZ-PCT-1

<160>   16

<170>   PatentIn version 3.5

<210>   1
<211>   62
<212>   PRT
<213>   Naja atra or Naja kaouthia

<400>   1

Leu Glu Cys His Asn Gln Gln Ser Ser Gln Thr Pro Thr Thr Thr Gly
1               5                   10                  15


Cys Ser Gly Gly Glu Thr Asn Cys Tyr Lys Lys Arg Trp Arg Asp His
                20                  25                  30


Arg Gly Tyr Arg Thr Glu Arg Gly Cys Gly Cys Pro Ser Val Lys Asn
            35                  40                  45


Gly Ile Glu Ile Asn Cys Cys Thr Thr Asp Arg Cys Asn Asn
        50                  55                  60


<210>   2
<211>   83
<212>   PRT
<213>   Naja atra or Naja kaouthia

<400>   2

Met Lys Thr Leu Leu Leu Thr Leu Leu Val Val Thr Ile Val Cys Leu
1               5                   10                  15


Asp Leu Gly Tyr Thr Leu Glu Cys His Asn Gln Gln Ser Ser Gln Thr
                20                  25                  30


Pro Thr Thr Thr Gly Cys Ser Gly Gly Glu Thr Asn Cys Tyr Lys Lys
            35                  40                  45


Arg Trp Arg Asp His Arg Gly Tyr Arg Thr Glu Arg Gly Cys Gly Cys
        50                  55                  60


Pro Ser Val Lys Asn Gly Ile Glu Ile Asn Cys Cys Thr Thr Asp Arg
65                  70                  75                  80

Cys Asn Asn


<210>    3
<211>    62
<212>    PRT
<213>    Naja atra or Naja kaouthia

<400>    3

Leu Glu Cys His Asn Gln Gln Ser Ser Gln Thr Pro Thr Thr Thr Gly
1                   5                   10                  15


Cys Ser Gly Gly Glu Thr Asn Cys Tyr Lys Lys Arg Trp Arg Asp His
            20                  25                  30


Arg Gly Tyr Arg Thr Glu Arg Gly Cys Gly Cys Pro Ile Val Lys Asn
            35                  40                  45


Gly Ile Glu Ser Asn Cys Cys Thr Thr Asp Arg Cys Asn Asn
            50                  55                  60


<210>    4
<211>    74
<212>    PRT
<213>    Bungarus multicinctus

<400>    4

Ile Val Cys His Thr Thr Ala Thr Ser Pro Ile Ser Ala Val Thr Cys
1                   5                   10                  15


Pro Pro Gly Glu Asn Leu Cys Tyr Arg Lys Met Trp Cys Asp Ala Phe
            20                  25                  30


Cys Ser Ser Arg Gly Lys Val Val Glu Leu Gly Cys Ala Ala Thr Cys
            35                  40                  45


Pro Ser Lys Lys Pro Tyr Glu Glu Val Thr Cys Cys Ser Thr Asp Lys
            50                  55                  60


Cys Asn Pro His Pro Lys Gln Arg Pro Gly
65                  70


<210>    5
<211>    74
<212>    PRT
<213>    Bungarus multicinctus

<400>    5

Ile Val Cys His Thr Thr Ala Thr Ile Pro Ser Ser Ala Val Thr Cys
1                   5                   10                  15

Pro Pro Gly Glu Asn Leu Cys Tyr Arg Lys Met Trp Cys Asp Ala Phe
        20              25              30

Cys Ser Ser Arg Gly Lys Val Val Glu Leu Gly Cys Ala Ala Thr Cys
        35              40              45

Pro Ser Lys Lys Pro Tyr Glu Glu Val Thr Cys Cys Ser Thr Asp Lys
        50              55              60

Cys Asn His Pro Pro Lys Arg Gln Pro Gly
65              70

<210> 6
<211> 72
<212> PRT
<213> Ophiophagus hannah

<400> 6

Ile Arg Cys Phe Ile Thr Pro Asp Val Thr Ser Gln Ala Cys Pro Asp
1           5               10              15

Gly Gln Asn Ile Cys Tyr Thr Lys Thr Trp Cys Asp Asn Phe Cys Gly
        20              25              30

Met Arg Gly Lys Arg Val Asp Leu Gly Cys Ala Ala Thr Cys Pro Thr
        35              40              45

Val Lys Pro Gly Val Asp Ile Lys Cys Cys Ser Thr Asp Asn Cys Asn
        50              55              60

Pro Phe Pro Thr Arg Glu Arg Ser
65              70

<210> 7
<211> 71
<212> PRT
<213> Ophiophagus hannah

<400> 7

Ile Arg Cys Phe Ile Thr Pro Asp Val Thr Ser Gln Ala Cys Pro Asp
1           5               10              15

Gly His Val Cys Tyr Thr Lys Met Trp Cys Asp Asn Phe Cys Gly Met
        20              25              30

Arg Gly Lys Arg Val Asp Leu Gly Cys Ala Ala Thr Cys Pro Lys Val
        35              40              45

```
Lys Pro Gly Val Asn Ile Lys Cys Cys Ser Arg Asp Asn Cys Asn Pro
    50              55              60

Phe Pro Thr Arg Lys Arg Ser
65              70


<210>   8
<211>   72
<212>   PRT
<213>   Ophiophagus hannah

<400>   8

Thr Lys Cys Tyr Lys Thr Gly Asp Arg Ile Ile Ser Glu Ala Cys Pro
1               5               10              15

Pro Gly Gln Asp Leu Cys Tyr Met Lys Thr Trp Cys Asp Val Phe Cys
            20              25              30

Gly Thr Arg Gly Arg Val Ile Glu Leu Gly Cys Thr Ala Thr Cys Pro
        35              40              45

Thr Val Lys Pro His Glu Gln Ile Thr Cys Cys Ser Thr Asp Asn Cys
    50              55              60

Asp Pro His His Lys Met Leu Gln
65              70


<210>   9
<211>   61
<212>   PRT
<213>   Naja kaouthia

<400>   9

Leu Glu Cys His Asn Gln Gln Ser Ser Gln Ala Pro Thr Thr Lys Thr
1               5               10              15

Cys Ser Gly Glu Thr Asn Cys Tyr Lys Lys Trp Trp Ser Asp His Arg
            20              25              30

Gly Thr Ile Ile Glu Arg Gly Cys Gly Cys Pro Lys Val Lys Pro Gly
        35              40              45

Val Asn Leu Asn Cys Cys Arg Thr Asp Arg Cys Asn Asn
    50              55              60


<210>   10
<211>   62
<212>   PRT
<213>   Naja kaouthia
```

14

<400> 10

```
Leu Glu Cys His Asn Gln Gln Ser Ile Gln Thr Pro Thr Thr Thr Gly
1               5               10                      15


Cys Ser Gly Gly Glu Thr Asn Cys Tyr Lys Lys Arg Trp Arg Asp His
            20                  25                  30


Arg Gly Tyr Arg Thr Glu Arg Gly Cys Gly Cys Pro Ser Val Lys Asn
        35                  40                  45


Gly Ile Glu Ile Asn Cys Cys Thr Thr Asp Arg Cys Asn Asn
    50                  55                  60
```

<210> 11
<211> 83
<212> PRT
<213> Naja kaouthia

<400> 11

```
Met Lys Thr Leu Leu Leu Thr Leu Leu Val Val Thr Ile Val Cys Leu
1               5               10                      15


Asp Leu Gly Tyr Thr Leu Glu Cys His Asn Gln Gln Ser Ser Gln Thr
            20                  25                  30


Pro Thr Thr Thr Gly Cys Ser Gly Gly Glu Thr Asn Cys Tyr Lys Lys
            35                  40                  45


Arg Trp Arg Asp His Arg Gly Tyr Arg Thr Glu Arg Gly Cys Gly Cys
        50                  55                  60


Pro Ser Val Lys Asn Gly Ile Glu Ile Asn Cys Cys Thr Thr Asp Arg
65                  70                  75                  80


Cys Asn Asn
```

<210> 12
<211> 60
<212> PRT
<213> Dendroaspis polylepis

<220>
<221> misc_feature
<222> (1)..(1)
<223> Xaa can be any naturally occurring amino acid

<400> 12

Xaa Ile Cys Tyr Asn His Gln Ser Thr Thr Arg Ala Thr Thr Lys Ser
1                       5                   10                  15

Cys Glu Glu Asn Ser Cys Tyr Lys Lys Tyr Trp Arg Asp His Arg Gly
                20                  25                  30

Thr Ile Ile Glu Arg Gly Cys Gly Cys Pro Lys Val Lys Pro Gly Val
            35                  40                  45

Gly Ile His Cys Cys Gln Ser Asp Lys Cys Asn Tyr
        50                  55                  60

<210>  13
<211>  60
<212>  PRT
<213>  Dendroaspis polylepis

<400>  13

Arg Ile Cys Tyr Asn His Gln Ser Thr Thr Arg Ala Thr Thr Lys Ser
1                       5                   10                  15

Cys Glu Glu Asn Ser Cys Tyr Lys Lys Tyr Trp Arg Asp His Arg Gly
                20                  25                  30

Thr Ile Ile Glu Arg Gly Cys Gly Cys Pro Lys Val Lys Pro Gly Val
            35                  40                  45

Gly Ile His Cys Cys Gln Ser Asp Lys Cys Asn Tyr
        50                  55                  60

<210>  14
<211>  72
<212>  PRT
<213>  Dendroaspis polylepis

<400>  14

Arg Thr Cys Asn Lys Thr Phe Ser Asp Gln Ser Lys Ile Cys Pro Pro
1                       5                   10                  15

Gly Glu Asn Ile Cys Tyr Thr Lys Thr Trp Cys Asp Ala Trp Cys Ser
                20                  25                  30

Arg Arg Gly Lys Ile Val Glu Leu Gly Cys Ala Ala Thr Cys Pro Lys
            35                  40                  45

Val Lys Ala Gly Val Gly Ile Lys Cys Cys Ser Thr Asp Asn Cys Asn
        50                  55                  60

Leu Phe Lys Phe Gly Lys Pro Arg

16

65          70

```
<210>  15
<211>  64
<212>  PRT
<213>  Bungarus fasclatus

<400>  15

Arg Ile Cys Leu Asn Gln Gln Gln Ser Thr Pro Glu Asp Gln Pro Thr
1               5               10              15


Asn Gly Gln Cys Tyr Ile Lys Thr Asp Cys Gln Asn Lys Thr Trp Asn
            20              25              30


Thr His Arg Gly Ser Arg Thr Asp Arg Gly Cys Gly Cys Pro Lys Val
        35              40              45


Lys Pro Gly Ile Asn Leu Arg Cys Cys Lys Thr Asp Lys Cys Asn Glu
        50              55              60


<210>  16
<211>  62
<212>  PRT
<213>  Bungarus fasclatus

<400>  16

Arg Ile Cys Leu Asn Gln Gln Ser Ser Glu Pro Gln Thr Thr Glu Thr
1               5               10              15


Cys Pro Asn Gly Glu Asp Thr Cys Tyr Asn Lys Thr Trp Asn Thr His
        20              25              30


Arg Gly Ser Arg Thr Asp Arg Gly Cys Gly Cys Pro Lys Val Lys Pro
        35              40              45


Gly Ile Asn Leu Arg Cys Cys Lys Thr Asp Lys Cys Asn Gln
        50              55              60
```

**Description**

**TECHNICAL FIELD**

The invention relates to a group of Elapidae postsynaptic neurotoxin monomer molecules that can inhibit the level of inflammatory cytokines in the brain tissue of the hippocampal region of Alzheimer's rats, and improve the learning and memory ability of Alzheimer's rats, and a method for treating Alzheimer's disease, belonging to the field of Biochemistry and biopharmaceutical areas.

**BACKGROUND OF TECHNOLOGY**

Senile dementia, also known as Alzheimer's disease (AD), is a senile disease characterized by progressive memory impairment, thinking and reasoning ability impairment, and movement disorder. Dementia is an acquired and persistent mental retardation syndrome caused by brain dysfunction, the incidence rate and prevalence of dementia are increasing with age. With the increasingly serious problem of the aging population, AD has become the fourth leading cause of death.

Though the pathogenesis of Alzheimer's disease is complex, the pathological basis commonly recognized by researchers at home and abroad is the degenerative lesion of the nervous system with brain cortical diffused atrophy which is accompanied by neuronal damage and death. In AD patients, the whole brain is diffusely atrophied, and neurologic lesions such as senile plaque (SP) with amyloid protein as the core, and neurofibrillary tangle with abnormal hyperphosphorylation of the tau protein as the core appear in the cerebral cortex and hippocampus. One theory holds that the neurotoxicity of the metabolites of amyloid precursor protein (APP) is the common pathway causing Alzheimer's disease, and the abnormal increase of its content in brain tissue may be the main factor inducing Alzheimer's disease. However, so far, clinical trials just aimed at eliminating tau protein have not been able to prove their real effectiveness in the treatment of Alzheimer's disease.

At the same time, there is also evidence that there is a strong focal inflammatory response in the brain of patients with Alzheimer's disease. There are activated microglia and astrocytes near the senile plaque. The study found that these activated cells can express interleukin cytokines such as inflammatory cytokines interleukin-1 β (interleukin-1 β, IL-1 β) and tumor necrosis factor-a(TNF-a) [1, 2]. Animal experimental studies have shown that amyloid peptide, the main component of senile plaques, can induce an inflammatory response that is similar to that of AD patients in the brain, accompanied by leukocyte activation and extravasation, as well as the expression of inflammatory cytokines. In addition, hyperphosphorylated amyloid can also cause an inflammatory reaction in nerve tissue. These studies confirmed that on top of the decreased expression of antiinflammatory cytokines (IL-1ra), The expression of other inflammatory cytokines such as TNF-a and IL-1 β in the brain of Alzheimer's patients is actually significantly increased [3,4]. Therefore, experts suggest that neuronal degeneration may be caused by inappropriate activation of immune and inflammatory responses in the brain. The super-strong immune response can attack neural tissue in the wrong direction, resulting in neuronal injury and death. So researchers point out that though the initiating factors of neuronal degeneration in patients are different, resulting in different outcomes of neuropathological damage, which, however, may be initiated by a similar process of cascade reaction of inflammatory cytokines, the massive release of inflammatory cytokines in the central nervous system may aggravate the production of amyloid peptide, the accumulation of amyloid peptide in blood vessels and the damage of brain white matter, resulting in a series of vicious cycles. In recent years, the discoveries through research in this field have confirmed the immune response of inflammatory factors near senile plaques in brain tissue, which leads to an increase in amyloid synthesis. Therefore, inhibiting excessive immune-inflammatory response has become another alternative attempt for the treatment of Alzheimer's disease.

From the public information, there are some reports on the treatment of senile dementia with cobra venom abroad, but cobra venom has a wide variety. The known components include neurotoxin, cytotoxin, cardiotoxin, nerve growth factor, hemolysin (DLP), CVA protein, membrane-active polypeptide, cobra venom factor, and other components, such as alkaline phosphomonoesterase, phosphodiesterase, acetylcholinesterase, L-amino acid oxidase, ribonuclease Protein hydrolases, etc. for cobra venom preparations, mixed toxins will pose life danger to organisms, which may be a strategy to enhance toxicity in the process of evolution. There are also synergistic effects between different types of toxins or similar toxin complexes in various snake venoms. The main toxins such as phospholipase A2 and three-finger toxin (neurotoxin) play an important role in the synergistic process [5,6]. It is this synergy that leads to fatal consequences for organisms; It has also been reported that cobra neurotoxin is used to treat Alzheimer's disease, however, there are many types of neurotoxins, in previous reports, the type of neurotoxin is not specified, and it's the first time that our invention has disclosed the use of Elapidae postsynaptic neurotoxin monomer molecule for the treatment of Alzheimer's disease.

## SUMMARY OF THE INVENTION

The invention discloses for the first time a group of Elapidae postsynaptic neurotoxin monomer molecules that can inhibit the increase of IL-1 beta and TNF-a levels in the hippocampal region of the senile dementia rat. In the Morris water maze experiment, the postsynaptic neurotoxin monomer molecule can significantly improve the learning and memory ability of senile dementia rats in the Morris water maze experiment (shortening evasion latency).

Our research finds that the Elapidae postsynaptic neurotoxin monomer molecule can inhibit inflammatory cytokines in the brain tissues of senile dementia rats and can improve the learning and memory ability of senile dementia rats. In addition to cobra postsynaptic neurotoxins, other species of Elapidae including Bungarus, King cobra, Black Mamba, Bungarus fasciatus, etc, their postsynaptic neurotoxins have the same function of inhibiting inflammatory cytokines in senile dementia rat brain tissues and improve the learning and memory ability of senile dementia rats, and this finding is also reported for the first time.

Synaptic refers to a structure in which an impulse of one neuron is transmitted to another neuron or another cell. The synapse is the part where the neuron is linked in terms of functioning as well as information transmission. Under an optical microscope, the axonal tip of one neuron can develop into multiple branches through a process of bifurcation, and finally, the tail end of each small branch is expanded to be in a cup shape or a spherical shape, which is called a synapse small body that may be in contact with the multiple cell bodies or dendrites of neurons to form a synapse. It can be seen from an electron microscope that the synapse is composed of a presynaptic membrane, a synapse gap, and a post-synaptic membrane.

Acetylcholine is a neurotransmitter that can specifically act on various choline receptors. Acetylcholine receptors include two types: muscarinic receptors and nicotinic receptors. The muscarinic type receptor is involved in parasympathetic nerve excitation effect, such as contraction of bronchial gastrointestinal smooth muscle, etc.; and the nicotinic receptor is located on the nerve node post-synaptic membrane or skeletal muscle endplate membrane, which can cause excitation of postpartum neurons of the autonomic ganglion or excitation of skeletal muscle.

The Elapidae neurotoxin can be divided into two categories according to different targeting sites: one type is a postsynaptic neurotoxin or an alpha-neurotoxin which is competitive with nicotinic acetylcholine receptors located at the nerve joint of the postsynaptic membrane to block the conduction of neurotransmitter acetylcholine [ 7 ], and the other class is a pre-synaptic neurotoxin or a beta-neurotoxin which acts directly on the motor neuron presynaptic membrane to block the release of acetylcholine so that the skeletal muscle loses a contraction function, thus paralyzed. Therefore, the neurotoxin (alpha-neurotoxin) of the cobra venom is an antagonist of the nicotinic acetylcholine receptor (nAChR) and binds to the nicotinic acetylcholine receptor of the neuron in antagonistic and slow reversible manner [ 8, 9 ]. Structurally, they have a common three-finger structure, and therefore are also referred to as three-finger toxins, the active part is close to the middle finger end [ 7 ], and the three-finger structure is multifunctional structure and can modulate acetylcholine and acetylcholine receptor [ 10 ].

The common ability to improve learning memory of senile dementia rats by Elapidae postsynaptic neurotoxin through inhibiting inflammatory responses is due to their common three-finger protein structure that can antagonize and regulate nicotinic acetylcholine receptors, and the latter is involved in the regulation of inflammatory responses [ 11, 12, 13, 14 ], which makes Elapidae postsynaptic neurotoxins identical and consistent in therapeutic efficacy. Therefore, by antagonizing and regulating the nicotinic acetylcholine receptor, the inflammatory response can be correspondingly inhibited, so the senile dementia rat's learning and memory abilities are improved, and thus the mechanism of Elapidae postsynaptic neurotoxin in the treatment of senile dementia. Our studies find that these postsynaptic neurotoxin monomer molecules are the neurotoxins with the mature proteins or polypeptides of amino acid sequence (FASTA) as follows:
Postsynaptic neurotoxins from Naja atra or Naja kaouthia

SEQ ID No.1 lechnqqssq tptttgcsgg etncykkrwr dhrgyrterg cgcpsvkngi eincttdrc nn
SEQ ID No.2 mktllltlllv vtivcldlgy tlechnqqss qtptttgcsg getncykkrw rdhrgyrter gcgcpsvkng ieincttdr cnn
SEQ ID No.3 lechnqqssq tptttgcsgg etncykkrwr dhrgyrterg cgcpivkngi esnccttdrc nn Postsynaptic neurotoxins from Bungarus multicinctus
SEQ ID No.4 ivchttatsp isavtcppge nlcyrkmwcd afcssrgkvv elgcaatcps kkpyeevtcc stdkcnphpk qrpg
SEQ ID No.5 ivchttatip ssavtcppge nlcyrkmwcd afcssrgkvv elgcaatcps kkpyeevtcc stdkcnhppk rqpg Postsynaptic neurotoxins from Ophiophagus hannah
SEQ ID No.6 lrcfitpdvt sqacpdgqni cytktwcdnf cgmrgkrvdl gcaatcptvk pgvdikccst dncnpfptre rs
SEQ ID No.7 lrcfitpdvt sqacpdghvc ytkmwcdnfc gmrgkrvdlg caatcpkvkp gvnikccsrd ncnpfptrkr s
SEQ ID No.8 tkcyktgdri iseacppgqd lcymktwcdv fcgtrgrvie lgctatcptv kpheqitccs tdncdphhkm lq Postsynaptic neurotoxins from Naja kaouthia
SEQ ID No.9 lechnqqssq apttktcsge tncykkwwsd hrgtiiergc gcpkvkpgvn lnccrtdrcn n
SEQ ID No.10 lechnqqsiq tptttgcsgg etncykkrwr dhrgyrterg cgcpsvkngi eincttdrc nn
SEQ ID No.11 mktllltlllv vtivcldlgy tlechnqqss qtptttgcsg getncykkrw rdhrgyrter gcgcpsvkng ieincttdr cnn Postsynaptic

neurotoxins from Dendroaspis polylepis

SEQ ID No.12 xicynhqstt rattksceen scykkywrdh rgtiiergcg cpkvkpgvgi hccqsdkcny

SEQ ID No.13 ricynhqstt rattksceen scykkywrdh rgtiiergcg cpkvkpgvgi hccqsdkcny

SEQ ID No.14 rtcnktfsdq skicppgeni cytktwcdaw csrrgkivel gcaatcpkvk agvgikccst dncnlfkfgk pr Postsynaptic neurotoxins from Bungarus fasclatus

SEQ ID No.15 riclnqqqst pedqptngqc yiktdcqnkt wnthrgsrtd rgcgcpkvkp ginlrccktd kcne

SEQ ID No.16 riclnqqsse pqttetcpng edtcynktwn thrgsrtdrg cgcpkvkpgi nlrccktdkc nq

Another advantage of the invention is product manufacturing, as the postsynaptic neurotoxin monomer molecule from Elapidae disclosed by the invention has a determined amino acid sequence, it can be produced through genetic engineering that solves the practical problem of scarcity of snake venom resources; even if we continue to process postsynaptic neurotoxin through separation and purification of natural snake venom, it is easier to control the quality and purity due to the determined amino acid sequence in the manufacturing process, which build up a necessary basis for the drug development of monomer from snake venom.

The present invention is further described below with reference to the attached drawings and specific embodiments. However, the following embodiments are not intended to limit the present invention. Meanwhile, the present disclosure and the like made according to the content disclosed in the present disclosure are all common substitutions in the art and are all within the protection scope of the present invention.

**BRIEF DESCRIPTION OF THE DRAWINGS:**

Fig-1 is a schematic diagram of 12 protein peaks separated by TSK/CM-650 (M) columns with ammonium acetate as buffer solution and eluent.

Fig- 2 is a line chart corresponding to the evasion latency of the senile dementia rat group, two isolated postsynaptic neurotoxin treatment groups, and the young rat control group (Table 1) before and after treatment.

Fig-3 is a line chart corresponding to the evasion latency (Table 2) of the senile dementia rat group, four recombinant postsynaptic neurotoxin treatment groups, and the young rat control group before and after treatment.

**METHOD OF IMPLEMENTATION**

Example A: Obtaining cobra neurotoxin polypeptide of SEQ ID NO: 1 and SEQ ID NO: 10.

The method comprises the following steps:

1. Separation and Purification of venom toxins from Naja atra

Dissolve 1g of Naja atra crude venom in 25ml 0.025 mol ph6 0 ammonium acetate buffer, centrifugation at low temperature, and take the supernatant; Use 0.025 mole ph6 0 ammonium acetate solution to balance TSK cm-650 (m) column; After loading the sample, 0.1 ~ 0.5 mol and 0.7 ~ 1.0 mol, pH5. 9 ammonium acetate buffer was used for elution in two-compartment gradient, and the UV detection parameter was set at 280nm; Elution flow rate: 48ml / h; Various toxin components were collected according to the recorded spectrum, and 12 protein peaks were washed out from the collected solution. (Fig-1)

2. Identifying the affinity of Naja atra toxin with nicotinic acetylcholine receptor (nAChR)

The method was based on the affinity test between 12 protein peaks and the nicotinic acetylcholine receptor (nAChR).

The principle of the affinity test

Because $\alpha$-bungarotoxin has a high affinity with the nicotinic acetylcholine receptor, the cobra neurotoxin can compete with $\alpha$-bungarotoxin for binding with the nicotinic acetylcholine receptor, and only these $\alpha$-bungarotoxin labeled with the radionuclide 1251 and bound with the nicotinic acetylcholine receptor will be precipitated and be counted by the GammaRadioimmuno assay Counters whilst unbound $\alpha$-bungarotoxin will be washed out. Therefore, 12 isolated proteins' binding inhibition rate against $\alpha$-bungarotoxin with nicotinic acetylcholine receptor (nAChR) could be used to measure the affinity between the isolated proteins and nicotinic acetylcholine receptor (nAChR), that's each isolated protein's bioactivity, which could be further determined by the $\gamma$ counting value per second (Bq) measured by Gamma-Radioimmuno assay Counters, and it becomes the index of proteins bioactivity.

The method for identifying cobra neurotoxin with high-affinity binding to the nicotinic acetylcholine receptor (nAChR) includes the following steps:
Take the above-isolated snake venom protein (just one purified of 12 protein peaks each time) and rat skeletal muscle nAChR extract, 1 μ L monoclonal antibody against acetylcholine nicotine receptor (mab35) 5.9mg/ml, labeled with radionuclide 125I α-Bungarotoxin 1 μ I(125I α- Btx)0.18 μ G / ml, after mixing, stand at 4 °C for more than 10 hours; The next day, Rabbit anti-rat IgG (4.5 mg/ml) was added for 10 μ L, stand at 4 °C for 2 hours; Centrifuge at 13000 rpm for 5 min, and wash the precipitate with Triton X-100 lotion for 3 times; γ count value per second (Bq) of protein bioactivity index was measured by GammaRadioimmuno assay Counters.

Calculation of "125I-αBtx-nAChR binding inhibitory rate (%)"

$$= 100 \times \frac{(CBSA-C\ cbx)}{(CBSA-C\alpha Btx)},$$

Where CaBtx, CBSA, and Ccbx refer to the bioactivity Bq values of positive control αBtx, negative control BSA, and isolated snake venom components respectively; taking α-bungarotoxin (αBtx) concentration (4μg/ml) as a positive control for inhibiting 125I-αBtx-nAChR binding, 100% inhibited, taking beef serum albumin (BSA)concentration (4μg/ml) as a negative control for inhibiting 125I-αBtx-nAChR binding, 0% inhibited.

The results show that there are 3 active peaks among 12 protein peaks, their inhibition rate can be up to 20 -50%, the protein peak with the highest inhibition rate is peak A, for which the amino acid sequencing is carried out.

3. The primary structure analysis of peak A protein namely amino acid sequencing

The method for amino acid sequence determination comprises the following steps. :

The peak A is purified and desalted with a reversed high-performance liquid chromatography (RP-HPLC) column (4.6 × 250 mm, Vydac RP-C8, 5 μM); and N-terminal and C-terminal amino acid sequences are determined by US ABI Company 491 protein sequence analyzer; BLSAT analysis is performed on the determined N-terminal amino acids sequence, and then work out the theoretical amino acid sequence of the Elapidae neurotoxin to be tested; analyzing the peptide fragment coverage rate of the Elapidae neurotoxin, and apply enzymolysis method on the protein supplied for the test by using trypsin, chymotrypsin and Glu-C enzyme; analyzing the enzymolysis peptide fragment sample by using LC-MS/MS (XUGG2 -XS QTOF Waters); and UniFi (1.8). 2 Waters) software is used for the analysis of LC-MS/MS data, and the peptide fragment coverage rate of the test product is determined according to the algorithm result. In addition, the sequence can be sequenced and confirmed by an Edman degradation method.

The amino acid sequence of the primary structure of the peak A protein is obtained by sequencing, the (FASTA form of SEQ ID NO. 1) is as follows:
lechnqqssq tptttgcsgg etncykkrwr dhrgyrterg cgcpsvkngi einccttdrc nn

By using the same separation and purification method, SEQ ID NO.10 postsynaptic neurotoxin of naja kouthia is obtained, the corresponding FASTA form of the amino acid sequence is as follows :

lechnqqsiq tptttgcsgg etncykkrwr dhrgyrterg cgcpsvkngi einccttdrc nn

Example B: Obtaining postsynaptic neurotoxin SEQ ID NO.4; SEQ ID NO.6; SEQ ID NO.12; SEQ ID NO. 16.
The above protein or polypeptide is obtained by gene recombinant technology and is specified as follows. :

1. Cloning of Recombinant Expression Vector

A DNA sequence is synthesized according to the gene of the postsynaptic neurotoxin SEQ ID NO: 4 provided on GenBank, PCR amplification is carried out on a target DNA sequence, a sequence encoding the intestinal kinase recognition site, and an NDE I enzyme digestion site is introduced at the 5 'end of the upstream primer, and a stop codon and a BamHI enzyme digestion site are introduced at the 5 'end of the downstream primer.

The gene containing the postsynaptic neurotoxin SEQ ID No. 4 is amplified by using a PCR method and cloned into a PBS-T vector, and the constructed recombinant sub-PBS-T-synaptic neurotoxin SEQ ID No. 4 shall be under examination of analysis and identification.

2. Protein expression in E coli.

The recombinant plasmid is transformed into the Escherichia coli expression vector pET15b, and the recombinant expression plasmid pET15b-postsynaptic neurotoxin SEQ ID No. 4 is constructed, and the correct recombinant transplanting to Escherichia coli BL21 (DE3) - LysS and examined under analysis and identification. The monoclonal antibody is inoculated into a 5G LB culture medium, cultured overnight at 37 DEG C, the day is inoculated into a 50 ml LB culture medium according to a ratio of 1: 100, and the culture is shaken at 37 DEG C until the OD600 nm is equal to 0.4 -0.6.

3. Collection and Analysis of Expressed Products

The transformation is continued with 1 mmol/L of IPTG for 3 hours, the expression bacteria, which means the induced and transformed Escherichia coli BL21 (DE3), is centrifuged, the inclusion body is dissolved in the buffer solution, and the supernatant and the precipitate are subjected to SDS-PAGE electrophoresis detection after centrifugation and collection, and the target protein exists in the form of inclusion bodies.

4. Affinity and Purification of Expressed Products

The inclusion body after ultrasonic breaking is dissolved in a buffer solution (6 mol/L of guanidine hydrochloride, 20 mmol/L of Tris-HCI, ph 8.0, 0.5 mol/L of Nacl, 5 mmol/L imidazole); the buffer solution is purified through a nickel-NTA column affinity chromatography, specifically, the buffer solution containing 20 mmol/L imidazole is washed to a baseline before loading, and finally, the buffer solution containing 300 mol/L imidazole is used for elution. The enterokinase is used to cut for obtaining the postsynaptic neurotoxin SEQ ID No. 4 protein.

5. Renaturation of Expressed Product

The eluted protein is dialyzed with 6 mol/L guanidine hydrochloride, 0.1 mol/L Tris-HCI-HCI Ph8.0, 0.01 mol LEDTA, 0.1 mmol/L PMSF, 10 mmol/L DTT buffer solution, the concentration of DTT and guanidine hydrochloride in the buffer solution is gradually decreased, and then the buffer solution is dialyzed with 10 - fold volume of 0.1 mol/L Tris-HCL Ph-8.0, 5 $\mu$mol/L CuSo4, and 20% glycerol. An RP-HPLC method is used for detecting the renaturation result, and through the comparison of retention time with a standard sample, the renaturation substance is identified, and the renaturation product is refrigerated and stored.

6. Amino Acid Sequence Determination

The peptide fragment coverage rate and the Edman degradation method are used for the analysis of the obtained neurotoxin, and the measured SEQ ID No. 4 sequence is compared with the amino acid sequence of the neurotoxin in the protein library, and after the confirmation of the consistency of the sequence, then the neurotoxin is used for the treatment of senile dementia rat in the next step.

The same recombinant method is used for obtaining the monomer molecule of Elapidae postsynaptic neurotoxin SEQ ID No. 6; SEQ ID NO. 12; and the SEQ ID NO. 16.

Example C: An experiment for improving the learning and memory ability of Alzheimer's disease rats by using Elapidae postsynaptic neurotoxin monomer molecule

1. Selection of Animal Model

According to the invention, a natural aging cognitive disorder (senile dementia) animal model is adopted, animal models of senile dementia are obtained through natural aging of animals, such as aged rats and the like, and nervous system changes such as cognitive impairment of the model are naturally occurring, and are closer to the real pathological changes of AD. Cummings reported the A$\beta$ precipitated plaque in the brain of aged canine accompanied by corresponding selective behavior impairment; A$\beta$ deposited in the forebrain substrate area was also reported by Higgins et al., and a memory defect [ 15 ] was detected.

2. Animals and Grouping

150 rats with age between 21 -22 months are adaptively fed for 10 days, the animals are free to take water, the room temperature is controlled to be 22 -25 DEG C, the humidity is 50 -70%, the illumination is 12 hours, and the dark is 12 hours. Rats (senile dementia rats) of cognitive disorders are screened through Morris water maze experiment training. The Morris water maze experiment is an experiment for forcing animals to swim and searching for an underwater platform, is mainly used for evaluating the animal space learning memory ability, is sensitive and reliable in experiment index, is simple and convenient to operate, and is a classical experiment for testing and evaluating senile dementia indexes of rats.

The water maze water depth is 50 cm, the water temperature is controlled at 22 -25 DEG C, and a platform is arranged in the pool center. The milk powder is put into the water tank and fully mixed until the water is milky white so that the rat cannot visually identify the position of the platform. During training days before formal testing, the rat, with the position facing to a wall, is put into water to find an evasion platform, if the rat finds the platform and stands for 3 seconds on the platform without slipping off, the training can be terminated, the time for reaching the platform and the distance are recorded, and the rat is allowed to stay for 10 seconds on the platform, thereby allowing the rat to learn memory. Those who can't find the platform continue to be recorded for 120 s, then guided to the platform and placed for 30 seconds, and the learning memory is trained. Training is carried out 4 times every day, the intervals between each training are about 15 -30 seconds, and rats get into water from four different directions, such training last for 4 days. The day 5th is the formal test day, the real-time image system automatically records the activity of the rat, and calculates the average time of the rat's first crossing platform (central region) to evaluate the learning memory ability of the animal. The upper limit value of 99% of the normal range of the average evasion latency of the rat (4 months old) is taken as a standard, and the old rat with an escape latency greater than 99% of the upper limit value is considered to be a known cognitive disorder old rat. Each rat is labeled so that the average evasion latency before treatment of each group of rats can be calculated after the completion of the last test.

The rats with cognitive disorder are screening out and randomly divided into two parts, and a part of the rats is randomly divided into three groups : ie, normal saline as senile dementia rat control group (non-drug administration, same volume saline for intragastric administration) and two isolated elapidae postsynaptic neurotoxin (SEQ ID NO. 1 and SEQ ID NO.10)as treatment group (the postsynaptic neurotoxin is prepared into a liquid form according to 45 μg/kg, continuously intragastric administration, 2 times per day, and 8 weeks of continuous administration), and meanwhile, a young rat control group (no drug, intragastric administration of same volume of physiological saline) is set up; and the another part is randomly divided into five groups: namely, senile dementia rat control group (no drug administration, intragastric administration of same volume physiological saline) and four recombinant elapidae postsynaptic neurotoxin (SEQ ID NO. 4.; SEQ ID NO. 6; SEQ ID NO.12; SEQ ID NO.16)as treatment group (the postsynaptic neurotoxin is prepared into a liquid form according to 45 μg/kg, continuously intragastric administration, 2 times per day, and 8 weeks of continuous administration); and meanwhile, a young rat control group (no drug, intragastric administration of same volume of physiological saline) is set up during experiment process, so that ten rats are kept for each group, and redundant rats are out of experimental group.

3. Behavior Test (Learning and Memory ability)

After 8 weeks of treatment followed by continuous training for 5 days, during which every day's average time of all groups of rats for finding a platform in water was recorded, and Morris water maze test is directly carried out on day 6. The time for finding a platform in water for each group of rats is tested. The treatment agent is still continuously administered during the training periods.

4. Experimental Results

Table-1 is the comparison of the average evasion latency among the senile dementia rat control group, two isolated purified postsynaptic neurotoxin treatment groups (SEQ ID NO.1 and SEQ ID NO.10), and the young rat control group.

Table-1

| (Second)X±S n = 10 | Before treatment | Day 1 | Day 2 | Day 3 | Day 4 | Day 5 | Day 6 |
|---|---|---|---|---|---|---|---|
| AD Control Group | 90 ±8.2 | 88 ±9.9 | 85 ±5.8 | 80 ±8.0 | 75 ±11.9 | 75 ±13.2 | 70 ±8.0 |
| SEQ ID NO.1 | 88 ±8.4 | 73 ±8.6 ** | 76 ±7.7 ** | 63 ±6.9 *** | 56 ±5.9 *** | 52 ±7.0 *** | 48 ±6.4 *** |
| SEQ ID NO.10 | 85 ±9.6 | 83 ±8.7 | 79 ±9.2 | 65 ±8.1 ** | 60 ±8.5 ** | 58 ±7.9 *** | 54 ±8.5 *** |
| Young Control Group | 28 ±5.8 ### | 23 ±4.1 ### | 21 ±4.0 ## | 18 ±3.1 ### | 19 ±3.6 ### | 16 ±2.8 ### | 16 ±3.4 ### |

Before administration of the treatment agent, there was no significant difference between the senile dementia rat control group and the two post-synaptic neurotoxin treatment groups, but there was a significant difference compared to the young rat control group, and # # # represents P < 0.001. After 8 weeks of administration of the treatment agent, the training for the Morris water maze experiment is performed again, from the first to fifth days, there was a significant difference between the senile dementia rat control group and some post-synaptic neurotoxin treatment groups, * * represents P < 0.01, * * * represents P < 0.001. There was a significant difference the day 6 between the senile dementia rat control group and the two post-synaptic neurotoxin treatment groups, * * * represents P < 0.001. # # # represents a significant difference among the young rat control group, the senile dementia rat control group, and two post-synaptic neurotoxin treatment groups, and P < 0.001 from the first to the sixth days. The data suggests that the Elapidae post-synaptic neurotoxin can improve the average evasion latency of the senile dementia rat group and improve the learning and memory ability of the senile dementia rat.

Table-2 is the comparison of average escape latency among the senile dementia rat control group, 4 recombinant postsynaptic neurotoxin treatment groups (SEQ ID NO.4; SEQ ID NO.6; SEQ ID NO.12; SEQ ID NO.16); and the young rat control group.

Table-2

| (Second)X±S n = 10 | Before treatment | Day 1 | Day 2 | Day 3 | Day 4 | Day 5 | Day 6 |
|---|---|---|---|---|---|---|---|
| AD Control Group | 88 ±8.7 | 88 ±9.9 | 85 ±11.2 | 81 ±8.0 | 77 ±11.9 | 75 ±12.1 | 73 ±8.0 |
| SEQ ID NO.4 | 90 ±8.4 | 82 ±10.5 | 73 ±8.9 * | 71 ±9.3 * | 58 ±7.8 ** | 56 ±8.1 ** | 53 ±7.3 *** |
| SEQ ID NO.6 | 92 ±9.0 | 72 ±8.7** | 72 ±7.10 ** | 68 ±8.2 ** | 59 ±8.3 ** | 50 ±6.8 *** | 44 ±6.7 *** |
| SEQ ID NO.12 | 86 ±8.5 | 79 ±9.3 | 70 ±9.5 ** | 72 ±7.9 * | 55 ±9.1 ** | 49 ±7.6 *** | 50 ±6.8 *** |
| SEQ ID NO.16 | 88 ±8.4 | 74 ±8.9** | 76 ±8.7 | 66 ±6.9 *** | 62 ±8.5 ** | 48 ±7.0 *** | 40 ±6.4 *** |
| Young Control Group | 31 ±5.8### | 26 ±4.1 ### | 23 ±4.0 ### | 20 ±3.1 ### | 19 ±3.6 ### | 18 ±2.8 ### | 16 ±3.4 ### |

There is no significant difference between the senile dementia rat control group and the 4 post-synaptic neurotoxin treatment groups before the administration of the treatment agent, but there is a significant difference between the above groups and the young rat control group, and # # # represents P < 0.001. After 8 weeks of administration of the treatment agent, the Morris water maze experiment training is performed again, there is a significant difference from the first to fifth day among the senile dementia rat control group and some post-synaptic neurotoxin treatment groups, * represents P < 0.05, * * represents P < 0.01, * * * represents P < 0.001. There is a significant difference on day 6 between the senile dementia rat control group and the 4 post-synaptic neurotoxin treatment groups, * * * represents P < 0.001.

# # # represents a significant difference among the young rat control group, the senile dementia rat control group, and the 4 post-synaptic neurotoxin treatment groups from the first to the sixth day, and P < 0.001

The data suggests that the Elapidae post-synaptic neurotoxins can improve the average evasion latency of the senile dementia rat group, thus improving the learning and memory ability of the senile dementia rat.

Example D: Determination of Inflammatory Cytokine in Brain Tissue of Experimental Rat 1 Method of Sampling and Testing

After completion of the experiment of Embodiment C, A group of rats comprising (the senile dementia rat control group, young rat control group, and the neurotoxin SEQ ID No. 1 treatment group) is intraperitoneally anesthetized with 10% hydrated chloral, and after completion of anesthesia, the three groups of experimental rats are sacrificed, and then the hippocampus tissue is rapidly separated on the operation table. According to 1g of the hippocampus tissue sample, normal saline of 4 °C, centrifugation at 13500 r/min, preparing homogenate with low-temperature for10 seconds each time, with interval of 30 seconds, continuous processing for 4 times, 10% tissue homogenate is ready, then low-temperature low-speed (4 °C and 3000 r/min) centrifugation for 15 min and the supernatant is taken and stored at-40 °C. The tests for the levels of the hippocampal region inflammatory cytokine interleukin-1 beta (interleukin-1 beta, IL-1 beta) and tumor necrosis factor-a(TNF-a) are operated according to the steps of the kit description of the ELISA method.

2. Experimental Results

Table-3 is the comparison of the levels of inflammatory cytokines interleukin-1 beta (IL-1 beta) and tumor necrosis factor-a(TNF-a) in the brain tissue of rat hippocampus among different groups.

Table-3

| n = 10 | IL-1β (ng/L,x±s) | TNF-a (ng/L,x±s) |
|---|---|---|
| AD Control Group | 44.60 ±5.57 | 60.70 ±6.72 |
| SEQ ID NO.1 Group | 36.40 ±3.93 ** | 51.20 ±7.05 ** |
| Young Control Group | 31.80 ±3.79 ### | 44.10 ±6.32 ### |

IL-1 beta and TNF-a levels of the young rat control group, compared with that of the AD control group, there is a significant difference between IL-1 beta and TNF-a, # # # represents $P < 0.001$; IL-1 beta and TNF-a levels of the treatment group of SEQ ID NO.1 compared with that of the AD control group, there is a significant difference between IL-1 beta and TNF-a, * * represents $P < 0.01$. The experimental data suggest that the Elapidae postsynaptic neurotoxin monomer molecule can reduce the neuronal damage of the hippocampal region by inhibiting the expression of inflammatory cytokines in the senile dementia rat hippocampal region brain tissue, which provides the pathological evidence for the improvement of learning and memory ability of the senile dementia rat.

The above embodiments are only illustrative of the embodiments and technical features of the present invention, and the purpose of the present invention is to enable the skilled person in the art to understand the contents of the present invention and are not to be construed as limiting the scope of protection of the present invention. Any equivalent change or modification made according to the spirit of the present invention shall fall within the protection scope of the present invention.

REFERENCES :

1. Mark Herre I et al.; Potential Related Biomarkrs in Alizheim's Disease. J Alzheimer's Disease 2005 8 (4) 369 -375.

2. GRIFFIN WWS. FLAVANITY AND NEURODEGENERATION DISEASE. Am. J Clin Nutr et al., 2006 83 (2): 470 S -474 S.

3. Wang et al.; Detection of Interleukin-1 and Tumorcellsis Factor-ain Serrum and Celanese Fluid Fluid in Patient with Alzheimer Disease Chin J Neuol, 2002 35 (6) 339 - 341.

4. Yinghua Shen. The activation of the nicotinic acetylcholine receptor is compared with the treatment of Alzheimer's disease. the 24th stage in the Pharmaceutical Journal of the Army Pharmacy.

5. Shengwei Xiong et al., Chunhong Huangang et al. Synergistic Strategy of Predomainant Toxins in SNAG Venoms. Toxiaecology Letters, 287 (2018) 142 -154.

6. Latisen et al., A H Toxin, Synergistic Gism in Snakke Venoms. Toxin Reviews, 35 (3-4), 165 -170 DOI: 10.1080/15569543.2016.

7. J White Hay et al., 1996 Snakke Neurotoxin. Human Neurology et al., 1996.

8. Naguib M et al Advances in Neurobiology of the Neuromucular Junction: Impact for the Anesthesiella. J AM, SOC. 96: 202 -31, 2002.

9. Abbas, M, Rahman S, Effects of alpha-7 Nicholic acetylcholine receptor positive allosteric modulator on lipoxycycline-induced neurodegeneration. EUR-J PHARMACOL. 783: 85 -91, 2016.

10. Pascale Marchot et al., The Three-Finger Toxin Fold: A Multifunctional Structural Scaffold Able to Modulate Cholergic Functions Journal of Neurochemistry, Vol. 142, Issue S2, 2017.

11. Guowu Zhou et al. ALPHA 7 NICOTINIC ACETYLCHOLINE RECEPTOR AND INFLAMMATION REGULATION. Modern Biomedical Progress 2009.

12. Zhigang Liu. Regulation of Alpha 7 nicotinic acetylcholine receptor with inflammation. Chinese Medical Guide 2014.

13. Hong Wang et al.;. Nicholic acetatyline receptor A 7 subunit IS AN ESSENTIAL REGULATOR OF INTAKE NATURE/VOL 421/23 January 2003.

14. Stingé Phanie St-Herre et al.;. Nicholic Acetatyline-Derived Victima-Derived Punicyte Hay-Derived Haplocyte Production and Survivale. PLOS Delta One/DOI: 10.1371/Journal. PONE 's DISEASE. 0150230 Februtary 29, 2016.

15. Tinghua Wang et al., Preparation of Neural Disease Animal Model Preparation Theory and Technology, Science Press 2005, ISBN II: 978 -7 -03 -043784 -6.

**Claims**

1. A method for treating senile dementia in a mammal. Said method comprising administering to a mammal in need thereof a pharmaceutical composition of a therapeutically effective amount of elapidae postsynaptic neurotoxin monomer molecule, and a pharmaceutically acceptable carrier base for use in reversing or relieving the symptoms of senile dementia.

2. A method for treating senile dementia in a mammal. Said method comprising administering to a mammal in need thereof a pharmaceutical composition of a therapeutically effective amount of elapidae postsynaptic neurotoxin monomer molecule, and a pharmaceutically acceptable carrier base for use in inhibiting or reducing the expression of interleukin-1 beta (IL-1 beta) and tumor necrosis factor-a (TNF-a) in the brain of senile dementia patients.

3. The elapidae neurotoxin of claims (1-2), wherein it is an elapidae postsynaptic neurotoxin polypeptide having the amino acid sequence shown in SEQ ID No.1 to SEQ ID No.16; or elapidae neurotoxin polypeptide homologues having 70% or more homology with the elapidae neurotoxin polypeptide of SEQ ID No.1 to SEQ ID No.16, and the biological function of the elapidae postsynaptic neurotoxin polypeptide homologues is the same as or similar to that of the elapidae neurotoxin polypeptide of the amino acid sequence ID No. 1 to SEQ ID No. 16.

4. Elapidae postsynaptic neurotoxin polypeptides or elapidae post synaptic neurotoxin polypeptides homologues according to claims (1-2), are further **characterized in that** they are derived from natural snake venoms, or synthesized from chemical polypeptides, or obtained from prokaryotic or eukaryotic hosts using recombinant technology ( such as Bacteria, yeast, higher plants, insects and mammalian cells).

5. The recombinantly produced elapidae postsynaptic neurotoxin polypeptide or its homologues according to claim (4), based on the host used in the recombinant production scheme, the polypeptide or its homologues of the present invention may be glycosylated, or may be non-glycosylated; Disulfide-bonded or non-disulfide-bonded. The polypeptides and its homologues described in the present invention may also include or exclude the starting methionine residue.

6. The elapidae postsynaptic neurotoxin polypeptide as in any of claims (1-5), further **characterized in that** the polypeptide in the present invention may include fragments of the above-mentioned various elapidae neurotoxin polypeptides after hydrolysis or enzymolysis, derivatives or analogs treated by physical, chemical or biological method, they are polypeptides which basically maintain the same biological function or activity as the above-mentioned elapidae neurotoxin polypeptide. The fragments, derivatives or analogs described in the present invention may be a polypeptide in which one or more amino acid residues are substituted, or a polypeptide having a substituent group in one or more amino acid residues, or combined with a compound (such as compounds that extend the half-life of a polypeptide, such as polyethylene glycol), or a polypeptide formed by fusion of a fatty chain, or a polypeptide formed by fusing an additional amino acid sequence to this polypeptide sequence. As described herein, these fragments, derivatives, and analogs are within the scope of those skilled in the art.

7. The method of claims (1-2) comprising intravenous, intramuscular, subcutaneous, intra-articular, oral, sublingual, nasal, rectal, topical, intradermal, intraperitoneal, intrathecal administration or transdermal administration.

8. The dose of elapidae postsynaptic neurotoxin of the method of claims (1-2) includes from 1 $\mu$g / Kg to 350 $\mu$g / kg each time, and the injection frequency ranges from once a day to multiple times a day, or multiple times a year.

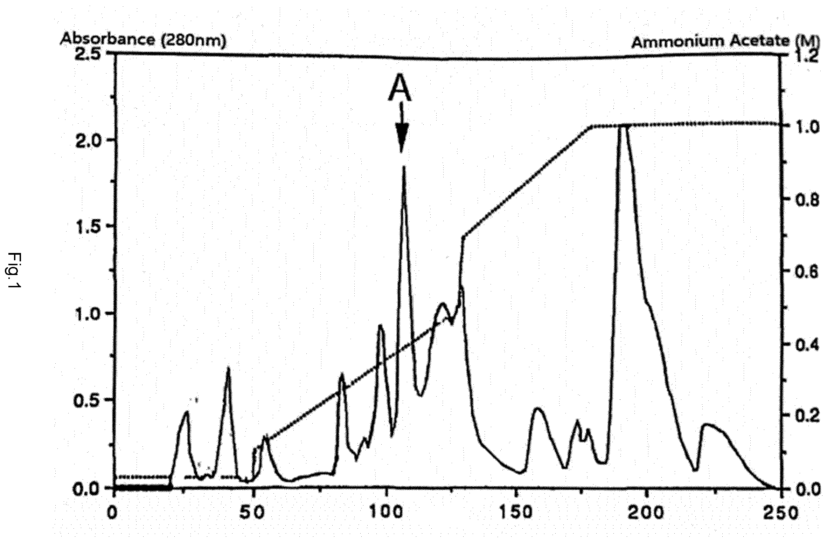

Fig.1

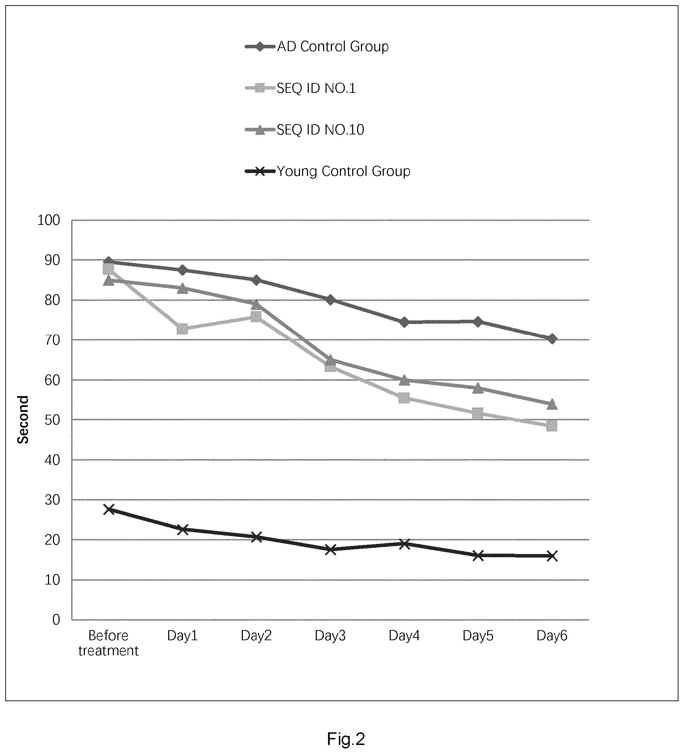

Fig.2

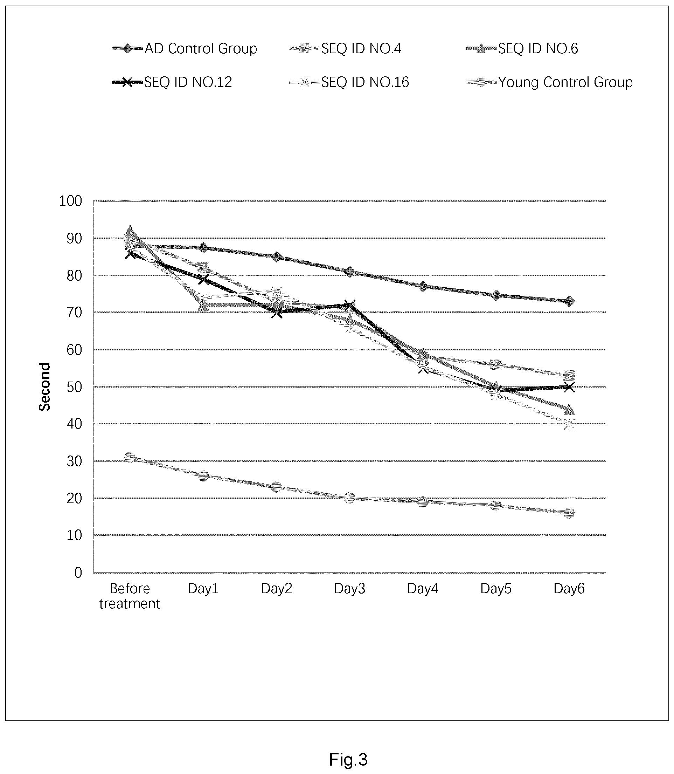

Fig.3

**TRANSLATION**

## INTERNATIONAL SEARCH REPORT

International application No.

**PCT/CN2020/000239**

| A. | CLASSIFICATION OF SUBJECT MATTER |
|---|---|

A61K 38/17(2006.01)i;  A61P 25/28(2006.01)i

According to International Patent Classification (IPC) or to both national classification and IPC

| B. | FIELDS SEARCHED |
|---|---|

Minimum documentation searched (classification system followed by classification symbols)

A61K,  A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , NCBI, baidu, blast, genbank, STNext, 祁展楷 , 祁海亚特 , 蛇, 眼镜蛇, venoms, 神经毒, toxin, neurotoxin, cobratoxin, three finger, 三指, 老年痴呆, 阿尔兹海默, Alzheimer, SEQ ID NOs: 1-16

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 111135288 A (QI, Zhankai) 12 May 2020 (2020-05-12)<br>entire document | 1-8 |
| PX | CN 111467479 A (QI, Zhankai) 31 July 2020 (2020-07-31)<br>entire document | 1-8 |
| X | CN 1064242 C (ANIMAL'S TOXIN AND SMAKE SOURCE DEVELOPMENT CENTRE, CHINESE ACADEMY OF SCIENCES) 11 April 2001 (2001-04-11)<br>description, page 6, paragraphs 1, 2 | 1, 2, 7, 8 |
| X | CN 102007142 A (MYOCEPT, INC.) 06 April 2011 (2011-04-06)<br>description, page 2 paragraph 12 - page 3 paragraph 14, page 5 paragraphs 37, 39, page 6 paragraphs 44-46, page 8 paragraph 57 - page 9 paragraph 60 | 3-6 |
| X | CHU, R.C. et al. "Cobrotoxin precursor (CBT) (Short neurotoxin 1)"<br>*UniProtKB/Swiss-Prot: P60770*, 15 June 2001 (2001-06-15),<br>entire document | 3-6 |
| X | QIN, C. et al. "short chain neurotoxin precursor [Naja atra]"<br>*GENBANK: AAG43384.1*, 02 January 2001 (2001-01-02),<br>entire document | 3-6 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 December 2020** | **12 January 2021** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2020/000239**

### C. DOCUMENTS CONSIDERED TO BE RELEVANT

| Category* | Citation of document, with indication, where appropriate, of the relevant passages | Relevant to claim No. |
|---|---|---|
| X | AGARD, D.A. et al. "PDB: 2ABX_A." *The Crystal Structure Of Alpha-Bungarotoxin At 2.5 Angstroms Resolution. Relation To Solution Structure And Binding To Acetylcholoine Receptor.*, 07 May 1986 (1986-05-07), entire document | 3-6 |
| X | CHIOU, S.H. et al. "UniProtKB/Swiss-Prot: P14613.1." *RecName: Full=Short neurotoxin 1; AltName: Full=Toxin C-6.*, 01 May 1992 (1992-05-01), entire document | 3-6 |
| X | LIU, C.S. et al. "UniProtKB/Swiss-Prot: P10808.1." *RecName: Full=Short neurotoxin 1; AltName: Full=Toxin V-II-1.*, 01 May 1992 (1992-05-01), entire document | 3-6 |
| X | TSAI, I.H. et al. "UniProtKB/Swiss-Prot: P0C555." *Neurotoxin 3FTx-RI precursor.*, 10 July 2007 (2007-07-10), entire document | 3-6 |
| A | CN 110090296 A (QI, Zhankai) 06 August 2019 (2019-08-06) claims 1-6, sequence table | 1-8 |

Form PCT/ISA/210 (second sheet) (January 2015)

**TRANSLATION**

**INTERNATIONAL SEARCH REPORT**

International application No.

**PCT/CN2020/000239**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a.  ☐  forming part of the international application as filed:

        ☐  in the form of an Annex C/ST.25 text file.

        ☐  on paper or in the form of an image file.

    b.  ☑  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2020/000239**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **1,2,7,8**
   because they relate to subject matter not required to be searched by this Authority, namely:

   [1] Claims 1, 2, 7, and 8 set forth a method for treating dementia in a patient, which belongs to treatment methods for diseases as stipulated in PCT Rule 39.1(iv). However, a search has nevertheless been carried out on the basis of a drug preparation use, that is, a use of an elapid snake postsynaptic neurotoxin monomer molecule in preparing a drug for treating dementia.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2020/000239**

| Patent document cited in search report | | | Publication date (day/month/year) | Patent family member(s) | | | Publication date (day/month/year) |
|---|---|---|---|---|---|---|---|
| CN | 111135288 | A | 12 May 2020 | None | | | |
| CN | 111467479 | A | 31 July 2020 | None | | | |
| CN | 1064242 | C | 11 April 2001 | CN | 1104096 | A | 28 June 1995 |
| CN | 102007142 | A | 06 April 2011 | WO | 2009105585 | A3 | 23 December 2009 |
| | | | | US | 9815875 | B2 | 14 November 2017 |
| | | | | WO | 2009105585 | A2 | 27 August 2009 |
| | | | | EP | 2250193 | B1 | 18 September 2019 |
| | | | | ES | 2757648 | T3 | 29 April 2020 |
| | | | | JP | 2011523935 | A | 25 August 2011 |
| | | | | CA | 2754352 | A1 | 27 August 2009 |
| | | | | IL | 207631 | D0 | 30 December 2010 |
| | | | | EP | 2250193 | A2 | 17 November 2010 |
| | | | | AU | 2009215436 | A1 | 27 August 2009 |
| | | | | US | 2011118190 | A1 | 19 May 2011 |
| CN | 110090296 | A | 06 August 2019 | WO | 2020057012 | A1 | 26 March 2020 |
| | | | | US | 2020093866 | A1 | 26 March 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARK HERRE I et al.** Potential Related Biomarkrs in Alizheim 's Disease. *J Alzheimer's Disease,* 2005, vol. 8 (4), 369-375
- GRIFFIN WWS. FLAVANITY AND NEURODEGENERATION DISEASE. *Am. J Clin Nutr,* 2006, vol. 83 (2), 470 S-474 S
- **WANG et al.** Detection of Interleukin-1 and Tumorcellsis Factor-ain Serrum and Celanese Fluid Fluid. *Patient with Alzheimer Disease Chin J Neuol,* 2002, vol. 35 (6), 339-341
- **YINGHUA SHEN.** The activation of the nicotinic acetylcholine receptor is compared with the treatment of Alzheimer's disease. *the 24th stage in the Pharmaceutical Journal of the Army Pharmacy*
- **SHENGWEI XIONG ; CHUNHONG HUANGANG et al.** Synergistic Strategy of Predomainant Toxins in SNAG Venoms. *Toxiaecology Letters,* 2018, vol. 287, 142-154
- **LATISEN et al.** A H Toxin, Synergistic Gism in Snakke Venoms. *Toxin Reviews,* vol. 35 (3-4), 165-170
- **J WHITE HAY et al.** *Snakke Neurotoxin. Human Neurology,* 1996
- **NAGUIB M et al.** Advances in Neurobiology of the Neuromucular Junction: Impact for the Anesthesiella. *J AM, SOC,* 2002, vol. 96, 202-31
- **ABBAS, M ; RAHMAN S.** Effects of alpha-7 Nicholic acetylcholine receptor positive allosteric modulator on lipoxycycline-induced neurodegeneration. *EUR-J PHARMACOL,* 2016, vol. 783, 85-91
- **PASCALE MARCHOT et al.** *The Three-Finger Toxin Fold: A Multifunctional Structural Scaffold Able to Modulate Cholergic Functions Journal of Neurochemistry,* 2017, vol. 142 (S2
- **GUOWU ZHOU et al.** ALPHA 7 NICOTINIC ACETYLCHOLINE RECEPTOR AND INFLAMMATION REGULATION. *Modern Biomedical Progress,* 2009
- **ZHIGANG LIU.** Regulation of Alpha 7 nicotinic acetylcholine receptor with inflammation. *Chinese Medical Guide,* 2014
- **HONG WANG et al.** Nicholic acetatyline receptor A 7 subunit. *IS AN ESSENTIAL REGULATOR OF INTAKE NATURE,* 23 January 2003, vol. 421
- **STINGÉ PHANIE ST-HERRE et al.** Nicholic Acetatyline-Derived Victima-Derived Punicyte Hay-Derived Haplocyte Production and Survivale. *PLOS Delta One,* 29 February 2016
- **TINGHUA WANG et al.** Preparation of Neural Disease Animal Model Preparation Theory and Technology. Science Press, 2005